(19) Europäisches Patentamt
European Patent Office
Office européen des brevets

(11) Veröffentlichungsnummer: **0 235 510**
**B1**

(12) # EUROPÄISCHE PATENTSCHRIFT

(45) Veröffentlichungstag der Patentschrift: **08.03.89**

(21) Anmeldenummer: **87100383.6**

(22) Anmeldetag: **14.01.87**

(51) Int. Cl.⁴: **C07D 311/72**, C07D 311/58 // C07C143/68, C07C121/16, C07C53/126, C07C31/125, C07C19/02

(54) **3,4-Dihydro-2,5,7,8-tetramethyl-2H-1-benzopyranderivate und Verfahren zu ihrer Herstellung.**

(30) Priorität: **23.01.86 US 821590**

(43) Veröffentlichungstag der Anmeldung: **09.09.87 Patentblatt 87/37**

(45) Bekanntmachung des Hinweises auf die Patenterteilung: **08.03.89 Patentblatt 89/10**

(84) Benannte Vertragsstaaten: **AT BE CH DE FR GB IT LI NL**

(56) Entgegenhaltungen:
**EP-A- 0 121 773**
**DE-A- 2 364 165**
**GB-A- 949 715**

(73) Patentinhaber: **F. HOFFMANN-LA ROCHE & CO. Aktiengesellschaft, CH-4002 Basel(CH)**

(72) Erfinder: **Cohen, Noal, 19 Euclid Place, Montclair, N.J.(US)**

(74) Vertreter: **Urech, Peter, Dr. et al, Grenzacherstrasse 124 Postfach 3255, CH-4002 Basel(CH)**

**EP 0 235 510 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft Halochromane, genauer genommen bezieht sich die Erfindung auf Verbindungen der Formel

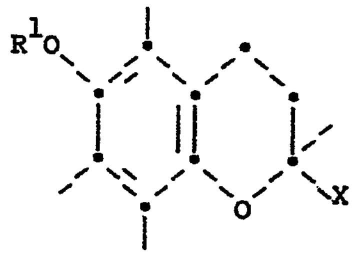

I

worin X Halogen und $R^1$ Methyl oder eine Hydroxy-Schutzgruppe, die durch basische Hydrolyse oder Hydrogenolyse abspaltbar ist, darstellen.

Die Erfindung betrifft weiterhin ein Verfahren zur Herstellung dieser neuen Verbindungen. Dieses Verfahren ist dadurch gekennzeichnet, dass man eine Verbindung der Formel

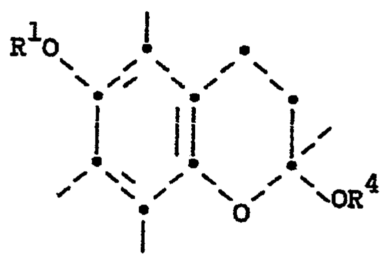

II

worin $R^1$ obige Bedeutung besitzt und $R^4$ Wasserstoff oder nieder Alkyl darstellt, mit einer Halogenwasserstoffsäure umsetzt.

Die Verbindungen der Formel I können als Zwischenprodukte für die Herstellung von Vitamin E oder bei der Herstellung von Zwischenprodukten für die Vitamin E-Herstellung Verwendung finden.

Zu diesem Zweck können die Verbindungen der Formel I beispielsweise mit einer Verbindung der Formel

$R^2Y$ III

worin Y ein Alkalimetall oder MgX darstellt, $R^2$ $-CH(COOR^3)_2$,

$$-CH_2-(CH_2)_2-\overset{\displaystyle CH_3}{\overset{|}{C}H}-(CH_2)_3-\overset{\displaystyle CH_3}{\overset{|}{C}H}-(CH_2)_3-\overset{\displaystyle CH_3}{\overset{|}{C}H}-CH_3$$

oder $-CH_2-CH=CH_2$ ist und $R^3$ nieder Alkyl ist und X Halogen darstellt, umgesetzt werden.

Bei der Umsetzung der neuen Verbindungen I mit einer Verbindung der Formel III bilden sich je nach der Bedeutung von $R^2$ die folgenden Verbindungen:

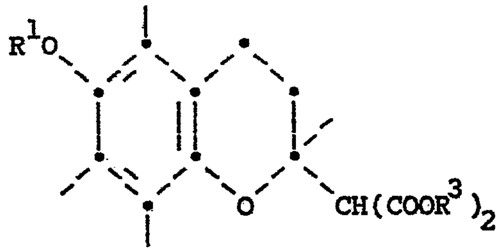

IV

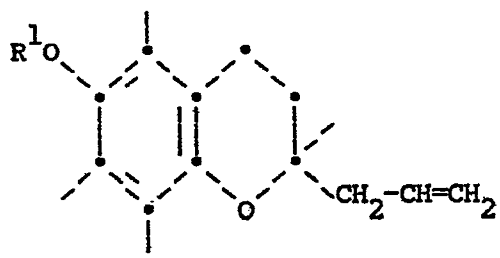

V; und

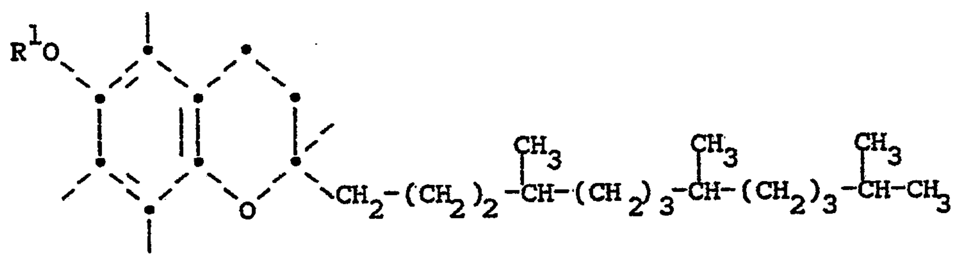

VI

worin $R^1$ obige Bedeutung besitzt und $R^3$ nieder Alkyl ist.

Wie oben gesagt, können die Verbindungen der Formel IV und V als Zwischenprodukte für die Herstellung von Vitamin E Verwendung finden, wobei die Verbindung der Formel VI der unmittelbare bekannte Precursor für Vitamin E darstellt.

Die neuen Verbindungen der Formel IV und V stellen einen weiteren Aspekt der vorliegenden Erfindung dar, und ein weiterer Aspekt betrifft die Herstellung der Verbindungen IV, V und VI und ihre Überführung in Vitamin E.

Im Rahmen der vorliegenden Erfindung bezieht sich der Begriff "Halogen" auf alle vier Halogenatome, also Fluor, Chlor, Brom und Jod, wobei Chlor, Brom und Jod bevorzugt sind und Chlor besonders bevorzugt ist. Weiterhin bezieht sich der Begriff "nieder Alkyl" auf geradkettige und verzweigte Alkylgruppen mit 1–7 Kohlenstoffatomen wie beispielsweise Methyl, Äthyl, Propyl, n-Butyl, Isopropyl, etc., wobei Methyl und Äthyl bevorzugt sind. Der Begriff "nieder Alkancarbonsäure" umfasst Alkancarbonsäuren mit 1–7 Kohlenstoffatomen, wie beispielweise Essigsäyre, Ameisensäure und Propionsäure.

Der Begriff "Aryl" bezieht sich zunächst auf mononukleare aromatische Kohlenwasserstoffreste wie Phenyl, welcher Rest unsubstituiert oder substituiert sein kann, und zwar ein- oder mehrfach substituiert mit nieder Alkylendioxy, Halogen, Nitro, nieder Alkyl oder nieder Alkoxy. Der Begriff umfasst aber auch polynukleare Arylreste wie Naphthyl. Auch diese Gruppen können unsubstituiert oder substituiert sein, wobei die Substituenten eine oder mehrere der oben genannten Gruppen sein können. Der bevorzugte Arylrest ist substituiertes oder unsubstituiertes Phenyl. Der Begriff "Aryl-nieder-alkyl" umfasst Aryl-nieder-alkylgruppen, worin Aryl und nieder Alkyl obige Bedeutung besitzen; bevorzugt sind diejenigen Gruppen, worin nieder Alkyl Methyl darstellt. Der bevorzugte Aryl-nieder-alkylsubstituent ist Benzyl. Der Begriff "Aryl-nieder-alkancarbonsäure" umfasst Aryl-nieder-alkancarbonsäuren, wobei Aryl und nieder Alkancarbonsäure wie oben definiert sind und demgemäss Arylcarbonsäuren umfasst. Die bevorzugte Aryl-nieder-alkancarbonsäure ist eine Arylcarbonsäure wie Benzoesäure.

Nieder Alkoxy umfasst nieder Alkoxygruppen mit 1–7 Kohlenstoffatomen, wie beispielsweise Methoxy und Äthoxy. Der Begriff "nieder Alkylendioxy" bezeichnet nieder Alkylendioxygruppen, die 2–7 Kohlenstoffatome aufweisen, ein Beispiel dafür ist der Äthoxylendioxyrest.

In der Verbindung der Formel II, also dem Ausgangsmaterial für die Herstellung der neuen Halochromane bzw. der Zwischenprodukte für Vitamin E, kann $R^1$ irgendeine Schutzgruppe darstellen, die durch basische Hydrolyse oder Hydrogenolyse abspaltbar ist. Zu diesem Zweck können alle die konventionellen Schutzgruppen, die durch basische Hydrolyse oder Hydrogenolyse abspaltbar sind, Verwendung finden. Beispiele solcher Schutzgruppen sind Estergruppen, abgeleitet von einer nieder Alkancarbonsäure oder einer Aryl-nieder-alkancarbonsäure. Zur Herstellung dieser (Verbindungen mit diesen) Esterschutzgruppen kann jede bekannte Methode Verwendung finden. Weiterhin kommen die konventionellen Ätherschutzgruppen in Betracht, wobei die bevorzugte Ätherschutzgruppe eine Arylmethylätherschutzgruppe wie einem Benzyläther darstellt. Die Abspaltung dieser Schutzgruppen ist dem Fachmann bekannt; es resultiert in jedem Fall eine Hydroxygruppe.

Wie oben gesagt, wird die Verbindung der Formel II durch Behandlung mit einer Halogenwasserstoffsäure in die Verbindung der Formel I übergeführt. Die bevorzugte Hydrohalogenwasserstoffsäure ist HCl, die Überführung geschieht zweckmässigerweise bei Temperaturen zwischen –30°C und +30°C, vorzugsweise zwischen –10°C und +10°C, zweckmässigerweise in einem inerten organischen Lösungsmittel. Im allgemeinen wird die Reaktion unter wasserfreien Bedingungen durchgeführt. Wie oben gesagt, arbeitet man vorzugsweise in einem inerten organischen Lösungsmittel. Zu diesem Zweck kann beispielsweise irgendein Äther Verwendung finden, ja dieser Äther ist sogar das bevorzugte Lösungsmittel. Als bevorzugte Äther kommen insbesondere Diäthyläther, Tetrahydrofuran, Glym und Diglym in Betracht.

Bei der Umsetzung der Formel I mit der Verbindung der Formel III entsteht, je nachdem, die Verbindung der Formel IV, V oder VI. Die Bildung der Verbindung IV, V oder VI hängt von dem Substituenten $R^2$ der Verbindung III ab.

Im Falle von Y = Alkalimetall kann Y ein konventionelles Alkalimetall sein, beispielsweise Lithium, Natrium oder Kalium, wobei Natrium und Kalium speziell bevorzugt sind. Die Reaktion der Verbindung der Formel I mit der Verbindung der Formel III (Y = Alkalimetall) wird zweckmässigerweise unter wasserfreien Bedingungen, zweckmässigerweise also in einem inerten organischen Lösungsmittel durchgeführt. Bei der Durchführung der Reaktion kann irgendein konventionelles inertes organisches Lösungsmittel Verwendung finden, wobei Äther, insbesondere die oben genannten Äther speziell gut geeignet sind. Das bevorzugte Lösungsmittel ist Diäthyläther. Man arbeitet bei der Reaktion bei Temperaturen zwischen –30°C und +30°C, wobei Temperaturen zwischen 0 und 15°C bevorzugt sind.

Im Falle von Y = MgX entsteht bei der besagten Reaktion die Verbindung IV, V oder VI, je nach Substituent $R^2$. Man arbeitet zweckmässigerweise in einem inerten organischen Lösungsmittel, zweckmässigerweise bei Temperaturen zwischen –100°C und 0°C. Ein bevorzugter, aber nicht ausschliesslicher, Bereich ist derjenige zwischen –80°C und –30°C. Die Reaktion kann in einem inerten organischen Lösungsmittel durchgeführt werden, wobei als solche Lösungsmittel wiederum Äther bevorzugt sind.

Die Überführung der Verbindung der Formel IV in die bekannten Vitamin E-Zwischenprodukte der Formel

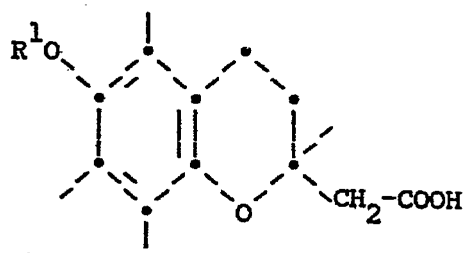

VII

worin $R^1$ obige Bedeutung besitzt,
kann mittels bekannter Methoden erfolgen. Jede konventionelle Methode die dazu dient, eine Malonsäure in eine Essigsäure überzuführen, kann hierbei Verwendung finden. Die bevorzugte Methode ist Hydrolyse, gefolgt von Decarboxylierung.

Die Verbindungen der Formel V können in die Verbindungen der Formel

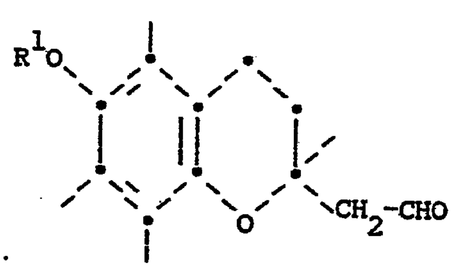

VIII

– also die bekannten Vitamin E-Zwischenprodukte –
worin $R^1$ obige Bedeutung besitzt,

übergeführt werden, indem von den bekannten Methoden, um Verbindungen mit terminalen Doppelbindungen in Aldehyde überzuführen, Gebrauch gemacht wird. Unter den bevorzugten Methoden rangiert die Ozonolyse, gefolgt von reduktiver Aufarbeitung.

In den unterstehenden Beispielen wurden die Reaktionen unter einer Argonatmosphäre durchgeführt. Im Falle der Kolonnenchromatographie wurde Silicagel 60 (Partikelgrösse: 0,063–0,2 mm) benützt. Der wasserfreie Äther und das wasserfreie Tetrahydrofuran wurden vor Gebrauch über Natriumbenzophenonketyl destilliert. Unter "Äther" wird im allgemeinen Diäthyläther verstanden.

Beispiel 1

rac-2-Chlor-3,4-dihydro-2,5,7,8-tetramethyl-6-(phenylmethoxy)-2H-1-benzopyran

Zu einer Lösung von 5 g (16 mMol) rac-3,4-Dihydro-6-(phenylmethoxy)-2,5,7,8-tetramethyl -2H-1-benzopyran-2-ol in 50 ml wasserfreiem Äther werden 5 g 4A Molekularsieb gegeben. Das Gemisch wird mechanisch gerührt, es befindet sich in einem Eisbad, und es wird HCl-Gas 30 Minuten durch die Lösung gespült. Man setzt das Rühren bei 0°C 30 Minuten lang fort und entfernt das Lösungsmittel im Vakuum. Der Rückstand wird mit 500 ml Hexan behandelt und die Lösung wird dekantiert. Die Hexan-Lösung wird hierauf mit 10 g wasserfreiem Calciumchlorid behandelt und es wird 2 Stunden weitergerührt. Die gebildeten Niederschläge werden filtriert, das Filtrat wird im Vakuum konzentriert, und zwar auf ein Volumen von ca. 20 ml. Mittels Kühlen auf –10°C und Rühren wird die Kristallisation eingeleitet, das Lösungsmittel wird im Vakuum abfiltriert, worauf 4,9 g (Ausbeute 92,8%) rac-2-Chlor-3,4-dihydro-2,5,7,8-tetramethyl-6-(phenylmethoxy) -2H-1-benzopyran als farbloser Niederschlag gebildet werden. Kolonnenchromatographie führt zur Zersetzung des Chlorchromans. Die Lagerung geschieht bei 0°C.

Beispiel 2

rac-2-Chlor-3,4-dihydro-2,5,7,8-tetramethyl-2H-1-benzopyran-6-ol-acetat

Ein Gemisch von 10 g (37,9 mMol) racemisches 3,4-Dihydro-6-acetyloxy-2,5,7,8-tetramethyl-2H-1-benzopyran -2-ol und 10 g 4A Molekularsieb in 200 ml wasserfreiem Äther werden unter Kühlung in einem Eisbad gerührt und es wird 30 Minuten HCl-Gas durchgeleitet. Hierauf wird das Gemisch filtriert und das Filtrat im Vakuum konzentriert. Der Niederschlag wird in 600 ml Hexan aufgenommen und es wird wasserfreies Calciumchlorid zugegeben. Das Gemisch wird 1 Stunde gerührt, filtriert, das Filtrat im Vakuum konzentriert, worauf 4,3 g (40,2% Ausbeute) racemisches 2-Chlor-3,4-dihydro-2,5,7,8-tetramethyl-2H-1-benzopyran -6-ol-acetat anfallen und zwar als farbloser Festkörper mit dem Schmelzpunkt 102–106°C; das Produkt verhält sich gegenüber Kolonnenchromatographie und gegenüber Dünnschichtchromatographie unstabil.

Beispiel 3

rac-3,4-Dihydro-2,5,7,8-tetramethyl-6-(phenylmethoxy)-2H-1-benzopyran-2-ylpropandisäure-dimethylester

Eine 370 mg-Probe (9,25 mMol) von 60% (Gew.-%) Natriumhydrid/40% (Gew.-%) Mineralöldispersion wird mittels Hexan ölfrei gewaschen und mit 20 ml wasserfreiem Tetrahydrofuran behandelt. Der resultierende Brei wird unter Eisbadkühlung gerührt und hierauf werden 1,056 g (8 mMol) Dimethylmalonat tropfenweise dazugegeben. Nach 10minütigem Rühren bei 0°C wird das Natriummalonat-Gemisch tropfenweise mit einer Lösung von rac-2-Chlor-3,4-dihydro-2,5,7,8-tetramethyl-6-(phenylmethoxy) -2H-1-benzopyran in 4 ml trockenem Tetrahydrofuran behandelt. Das Gemisch wird 1,5 Stunden bei 0°C weitergerührt, hierauf das Reaktionsgemisch in Wasser gegeben und dreimal mit Äther extrahiert. Die ätherischen Extrakte werden mittels Magnesiumsulfat getrocknet, filtriert und im Vakuum konzentriert, was zu einem gelben öligen Niederschlag führt. Dieses Material wird in 5 ml Petroläther gelöst (30–60°C) und gerührt, was zu Bildung eines weissen Niederschlags führt. Der Niederschlag wird durch Filtration isoliert und aus Petroläther umkristallisiert, wobei 0,4 g (23,4% Ausbeute) rac-3,4-Dihydro-2,5,7,8-tetramethyl-6-(phenylmethoxy)-2H -1-benzopyran-2-ylpropandisäure-dimethylester anfallen. Das Produkt ist farblos und weist einen Schmelzpunkt von 80–81°C auf.

Beispiel 4

rac-3,4-Dihydro-6-(phenylmethoxy)-2-(2-propenyl)-2,5,7,8-tetramethyl-2H-1-benzopyran

Zu 12 ml (24 mMol) 2M Allylmagnesiumchlorid in Tetrahydrofuran wird unter Kühlung in einem Eisbad unter Rühren eine Lösung von 4,95 g (14,98 mMol) racemischem 2-Chlor-3,4-dihydro-2,5,7,8-tetramethyl-6-(phenylmethoxy) -2H-1-benzopyran in 60 ml wasserfreiem Äther gegeben. Das Reaktionsgemisch wird 6 Stunden bei 0°C gerührt und hierauf aufgearbeitet, nämlich auf Eis gegeben, mit gesättigter Ammonchloridlösung behandelt und hierauf mit Äther extrahiert. Das gebildete Produkt (5,4 g eines hellgelben Öls) wird in 40 ml Methanol gelöst und hierauf mit 10 ml Äther, die 10 mg p-Toluolsulfonsäure-monohydrat enthalten, versetzt. Das Gemisch wird bei Zimmertemperatur 21 Stunden gerührt und hierauf im Vakuum konzentriert. Der Niederschlag wird mittels 75 g Silicagel chromatographiert. Die Elution mit Hexan-Äther (40:1, Volumenteile) liefert 2,88 g (Ausbeute 57,2%) rac-3,4-Dihydro-6-(phenylmethoxy)-2-(2-propenyl)-2,5,7,8-tetramethyl-2H-1-benzopyran als farbloses Öl.

Beispiel 5

(4R,8R)-1-Brom-4,8,12-trimethyltridecan

Eine Lösung von 10,44 g (45,8 mMol) (3R,7R)-3,7,11-Trimethyldodecan-1-ol in 150 ml wasserfreiem Pyridin werden unter Aceton-Eis-Kühlung gerührt, währenddem 17,4 g (91,3 mMol) p-Toluolsulfonylchlorid in einer Portion zugegeben werden. Das Gemisch wird in einem Aceton-Eisbad 2 Stunden gerührt, hierauf 40 Stunden bei 0°C gehalten, bevor 300 ml Eis-Wasser-Gemisch zugegeben werden. Das Produkt wird mit dreimal 300 ml Äther extrahiert. Die ätherischen Extrakte werden kombiniert, mit 400 ml kalter 3N Salzsäure/gesättigter Kochsalzlösung gewaschen, hierauf mittels Magnesiumsulfat getrocknet, filtriert und im Vakuum konzentriert. Es fallen 16,6 g (94,9%) (3R,7R)-3,7,11-Trimethyldodecyl-p-toluolsulfonat an.

Ein Gemisch von (43,45 mMol) obigen Tosylats, 4,25 g (86,7 mMol) Natriumcyanid in 80 ml Äthanol und 20 ml Wasser werden gerührt und 2,5 Stunden bei Rückflusstemperatur gehalten. Der Grossteil des Äthanols wird im Vakuum entfernt und der Rückstand mit 75 ml Wasser und 75 ml gesättigter Kochsalzlösung behandelt, hierauf dreimal mit 100 ml Äther extrahiert. Die ätherischen Extrakte werden kombiniert, mit gesättigter Kochsalzlösung gewaschen, mittels Magnesiumsulfat getrocknet, durch Silicagel filtriert und im Vakuum konzentriert. Man erhält 10,25 g (99,5%) (4R,8R)-4,8,12-Trimethyltridecannitril als gelbes Öl. Eine gaschromatographische Analyse signalisiert eine Reinheit von 95,2% an.

Ein Gemisch dieses Nitrils (43,25 mMol), 18,4 g (0,33 Mol) Kaliumhydroxid in 162 ml Äthylenglykol und 13,5 ml Wasser wird in einem Ölbad bei 150°C gerührt, und zwar während 4 Stunden, hierauf wird auf 0–5°C abgekühlt und in 300 ml 6N Salzsäure gegeben. Das Gemisch wird hierauf mit zweimal 400 ml Äthylacetat extrahiert. Die organischen Extrakte werden kombiniert, mit 300 ml gesättigter Kochsalzlösung gewaschen, über Magnesiumsulfat getrocknet, filtriert und im Vakuum konzentriert, worauf 11,1 g (100%) (4R,8R)-4,8,12-Trimethyltridecancarbonsäure als Öl anfallen.

Eine Lösung dieser Säure in 50 ml Toluol wird bei Zimmertemperatur gerührt, währenddem 25 ml Natrium-bis(2-methoxyäthoxy)aluminiumhydrid in Toluol tropfenweise zugegeben werden. Nach 3stündigem Rühren bei Zimmertemperatur wird das Gemisch durch vorsichtige Zugabe von 5 ml Äthanol zersetzt. Das Gemisch wird mit 300 ml 6N Salzsäure behandelt und mit dreimal 300 ml Äthylacetat extrahiert. Die organischen Extrakte werden kombiniert, mit 300 ml gesättigter Kochsalzlösung gewaschen, mit Magnesiumsulfat getrocknet, filtriert und im Vakuum konzentriert. Die Kugelrohrdestillation (Badtemperatur: 160°C, Druck 1 mmHg) des Niederschlages (9,7 g) liefert 7,0 g (4R,8R)-4,8,12-Trimethyltridecanol als farblose Lösung, eine gaschromatographische Analyse signalisiert eine Reinheit von 94,5%. Der Destillationsrückstand enthält nicht umgesetztes Ausgangsmaterial (Säure) und wird nochmals mit 6 ml Natrium-bis(2-methoxäthoxy)aluminiumhydrid reduziert, wobei obige Bedingungen eingehalten werden. Auf diese Weise entstehen nochmals 1,8 g des Alkohols mit einer Reinheit von 95,8%. Die Totalausbeute beträgt demgemäss 8,8 g (84,2%).

Zu einer Lösung von 9,6 g (39,6 mMol) (4R,8R)-4,8,12-Trimethyltridecanol in 30 ml wasserfreiem N,N-Dimethylformamid werden 10,7 g (40,84 mMol) Triphenylphosphin zugegeben. Das Gemisch wird in einem Aceton-Eis-Bad bei –10°C gerührt, währenddem 2,1 ml (41 mMol) Brom tropfenweise zugegeben werden. Die Temperatur steigt auf 5°C an. Das Reaktionsgemisch wird bei Zimmertemperatur gerührt, und zwar 1 Stunde, hierauf wird zu 100 ml Wasser und 150 ml Hexan gegeben. Nach Filtration werden die Schichten getrennt und die wässrige Phase wird zweimal mit 150 ml Hexan extrahiert. Die Hexanschichten werden mit gesättigter $NaHCO_3$-Lösung gewaschen, mit Magnesiumsulfat getrocknet, durch einen Pfropfen von Silicagel filtriert und schliesslich im Vakuum konzentriert. Eine Kugelrohrdestillation bei 150–160°C Badtemperatur und einem Druck von 1 mm Quecksilbersäure liefert zwei Fraktionen: 5,15 g eines Materials mit 95,8% Reinheit (gemäss Gaschromatographie) und 3,55 g eines Materials von 97,9% Reinheit; die Ausbeute beträgt 72,2%. Die Redestillation der grösseren Fraktion liefert das (4R,8R)-1-Brom-4,8,12-trimethyltridecan als farblose Lösung mit einem Siedepunkt von 120°C bei 0,15 mmHg und in einer Reinheit von 97,9%; [alpha] $^{25}_{D}$ = –3,01°C (c 2,09, Hexan).

Beispiel 6

(2RS,4′R,8′R)-alpha-Tocopheryl-benzyläther

Eine Grignard-Lösung wird hergestellt aus 0,28 g (11,2 mMol) Magnesium und 3,4 g (11,2 mMol) (4R,8R)-1-Brom-4,8,12-trimethyltridecan in 25 ml wassserfreiem Äther. Die Grignard-Reaktion wird gestartet durch Zugabe weniger Tropfen von 1,2-Dibromäthan, und das Gemisch wird nun 3,5 Stunden unter Rühren auf Rückflusstemperatur erhitzt. Diese $C_{16}$-Grignard-Lösung wird hierauf tropfenweise und unter Rühren zu einer Lösung von 2,5 g (7,87 mMol) rac-2-Chlor-3,4-dihydro-2,5,7,8-tetramethyl-6-(phenylmethoxy) -2H-1-benzopyran in 25 ml wasserfreiem Äther gegeben, dann wird auf –10°C abgekühlt (Eis-Aceton-Bad). Das resultierende Gemisch wird 18 Stunden bei 0°C gerührt und hierauf mit 100 ml gesättigter Ammoniumchloridlösung behandelt. Das Produkt wird durch zweimalige Extraktion mit je 100 ml Äther isoliert. Die ätherischen Extrakte werden mit gesättigter Kochsalzlösung gewaschen, mittels Magnesiumsulfat getrocknet, filtriert und im Vakuum konzentriert. Der Rückstand (4,95 g) wird in 50 ml Methanol gelöst und es werden 30 ml Äther mit einem Gehalt von 200 mg p-Toluolsulfonsäure-monohydrat zugegeben. Man rührt 24 Stunden bei Zimmertemperatur, konzentriert die Lösung im Vakuum und chromatographiert den Rückstand auf 200 g Silicagel. Eine Elution mit Hexan-Äther (40:1 Volumenteile) liefert 1,82 g (44,5%) reinen (2RS,4'R,8'R)-alpha-Tocopheryl-benzyläther. Die Identität dieses Materials wird unter Zuhilfenahme einer authentischen Probe durch die spektralen Daten und Dünnschichtchromatographie etabliert.

Beispiel 7

rac-2-Chlor-3,4-dihydro-2,5,7,8-tetramethyl-6-(phenylmethoxy)-2H-1-benzopyran

Ein Gemisch von 10 g rac-2-Methoxy-3,4-dihydro-2,5,7,8-tetramethyl-6-(phenylmethoxy)-2H-1-benzopyran, 10g Calciumchlorid, 100 ml Hexan und 50 ml Diäthyläther werden 1 Stunde bei –5 bis –10°C gerührt, währenddem Salzsäuregas durchgeleitet wird. Eine zusätzliche Menge von 10 g Calciumchlorid wird zugegeben und es wird 2 Stunden bei Zimmertemperatur weitergerührt. Das Gemisch wird filtriert, das Filtrat im Vakuum konzentriert, wobei 10,2 g des gewünschten Materials als hellbraunes Öl anfallen. Proton-NMR-Analyse zeigt, dass das Produkt zu 66% aus dem gewünschten Material und zu 33% aus dem Ausgangsprodukt 2-Methoxychroman besteht.

Beispiel 8

rac-3,4-Dihydro-2,5,7,8-tetramethyl-6-(phenylmethoxy)-2H-1-benzopyran-2-essigsäure

Ein Gemisch von 0,43 g (1 mMol) rac-3,4-Dihydro-2,5,7,8-tetramethyl-6-(phenylmethoxy)-2H-1 -benzopyran-2-ylpropandisäure-dimethylester, 0,56 g (10 mMol) Kaliumhydroxyd und 25 ml Äthylenglykol-Wasser (Verhältnis 9:1) werden unter Rühren 8 Stunden bei Rückflusstemperatur gehalten. Das Gemisch wird gekühlt, mit Wasser verdünnt, mit Äther extrahiert und der Ätherextrakt verworfen. Die wässrige alkalische Lösung wird mit 3N Salzsäure angesäuert und das saure Produkt durch ätherische Extraktion isoliert. Die ätherischen Extrakte werden kombiniert, mit Wasser und Natriumchlorid gewaschen, mit Magnesiumsulfat getrocknet, filtriert und im Vakuum konzentriert. Eine Umkristallisation des Niederschlages des wässrigen Äthanols liefert rac-3,4-Dihydro-2,5,7,8-tetramethyl-6-(phenylmethoxy)-2H-1 -benzopyran-2-essigsäure, die nun gemäss Helv. Chim Acta 61, 837 (1978); Helv. Chim. Acta 59, 290 (1976) und Helv. Chim. Acta 64, 1158 (1981) in Vitamin E übergeführt werden können.

Beispiel 9

rac-3,4-Dihydro-2,5,7,8-tetramethyl-6-(phenylmethoxy)-2H-1-benzopyran-2-acetaldehyd

Ein Gemisch von Ozon und Sauerstoff wird in eine gerührte Lösung von 0,67 g (2 mMol) rac-3,4-Dihydro-2,5,7,8-tetramethyl-2-(2-propenyl)-6 -(phenylmethoxy)-2H-1-benzopyran in 100 ml Methanol eingeleitet, wobei die Temperatur bei –78°C gehalten wird. Nachdem das olefinische Ausgangsmaterial konsumiert ist, wird der Ozonfluss gestoppt und die Lösung mit einem Überschuss von Dimethylsulfid behandelt. Hierauf lässt man sie Zimmertemperatur annehmen. Die Lösung wird im Vakuum konzentriert. Der Rückstand wird auf Silicagel chromatographiert und zum rac-3,4-Dihydro-2,5,7,8-tetramethyl-6-(phenylmethoxy)-2H-1 -benzopyran-2-acetaldehyd zu gelangen, welches Material gemäss Helv. Chim. Acta (siehe Beisiel 8) in Vitamin E übergeführt werden kann.

Beispiel 10

(2RS,4'R,8'R)-alpha-Tocopherol

Eine Probe von 0,6 g (2,13 mMol) rac-2-Chlor-3,4-dihydro-2,5,7,8-tetramethyl-2H-1-benzopyran -6-ol-acetat wird mit (4R,8R)-4,8,12-Trimethyltridecylmagnesiumbromid behandelt, wie dies gemäss Beispiel 6 oben beschrieben ist; es wird in diesem Beispiel jedoch ein Überschuss an Grignard-Reagens eingesetzt. Die Chromatographie des rohes Produktes an Silicagel liefert das (2RS,4'R,8'R)-alpha-Tocopherol als Öl.

Beispiel 11

(2RS,4'R,8'R)-alpha-Tocopheryl-methyläther

Gemäss Beispiel 6 wird rac-2-Chlor-6-methoxy-3,4-dihydro-2,5,7,8-tetramethyl -2H-1-benzopyran mit (4R,8R)-4,8,12-Trimethyltridecylmagnesiumbromid behandelt, worauf der (2RS,4'R,8'R)-alpha-Tocopheryl-methyläther als Öl anfällt.

Beispiel 12

(2RS,4'R,8'R)-alpha-Tocopherol

Eine Lösung von 0,25 g (0,56 mMol) 2RS,4'R,8'R)-alpha-Tocopheryl-methyläther in 10 ml 1,2-Dichloräthan wird mit 0,7 g (2,25 mMol) Bortribromid-dimethylsulfidkomplex behandelt und das Reaktionsgemisch wird 20 Stunden bei Zimmertemperatur gerührt. Hierauf wird das Gemisch gekühlt und mit Wasser behandelt. Die organische Lösung wird abgetrennt, mit Wasser und mit Kochsalzlösung gewaschen, über Magnesiumsulfat getrocknet, filtriert und im Vakuum konzentriert. Der Rückstand wird an Silicagel chromatographiert und es fällt das (2RS,4'R,8'R)-alpha-Tocopherol als Öl an.

Beispiel 13

rac-6-Methoxy-3,4-dihydro-2,5,7,8-tetramethyl-2H-1-benzopyran-2-ol

Ein Gemisch von 84,3 g (0,337 Mol) rac-2,6-Dimethoxy-3,4-dihydro-2,5,7,8-tetramethyl-2H-1 -benzopyran, 370 ml Aceton, 300 ml Wasser und 2,5 ml konzentrierte Salzsäure wird solange destilliert, bis die Destillationstemperatur 90°C erreicht. Nach Kühlen wird das Gemisch mit Wasser verdünnt und dreimal mit Äther extrahiert. Die ätherischen Extrakte werden kombiniert, mit gesättigter Kochsalzlösung gewaschen, über Magnesiumsulfat getrocknet, filtriert und schliesslich im Vakuum konzentriert. Der Rückstand wird aus wässrigem Aceton umkristallisiert, um zum rac-6-Methoxy-3,4-dihydro-2,5,7,8-tetramethyl-2H-1-benzopyran -2-ol zu gelangen, welches Produkt ein weisslicher Festkörper darstellt und in Mengen von 34,6 g (43,5%) und 11,1 g (13,9%) anfällt.

Beispiel 14

rac-2-Chlor-6-methoxy-3,4-dihydro-2,5,7,8-tetramethyl-2H-1-benzopyran

Gemäss Verfahren des Beispiels 1 wird das rac-6-Methoxy-3,4-dihydro-2,5,7,8-tetramethyl-2H-1-benzopyran -2-ol in das rac-2-Chlor-6-methoxy-3,4-dihydro-2,5,7,8-tetramethyl-2H-1-benzopyran übergeführt. Auch dieses Material stellt einen weisslichen Festkörper dar; die Ausbeute beträgt 90,2%.

Beispiel 15

rac-3,4-Dihydro-2,5,7,8-tetramethyl-6-methoxy-2H-1-benzopyran-2-ylpropandisäure-diäthylester

Gemäss Verfahren des Beispiels 3 wird rac-2-Chlor-6-methoxy-3,4-dihydro-2,5,7,8-tetramethyl-2H-1-benzopyran zum rac-3,4-Dihydro-2,5,7,8-tetramethyl-6-methoxy-2H-1-benzopyran-2-ylpropandisäure-diäthylester umgesetzt [wobei aber anstelle von Dimethylmalonat Diäthylmalonat eingesetzt wird]. Das Produkt stellt einen farblosen Festkörper vom Schmelzpunkt 74–76°C dar. Die Ausbeute beträgt 54% und die Reinigung wird durch eine Kombination von HPLC und Umkristallisieren aus Petroläther bewerkstelligt.

Beispiel 16

rac-3,4-Dihydro-6-methoxy-2,5,7,8-tetramethyl-2H-1-benzopyran-2-essigsäure

rac-3,4-Dihydro-6-hydroxy-2,5,7,8-tetramethyl-2H-1-benzopyran-2-essigsäure

Ein Gemisch von 1,29 g (3,41 mMol) rac-3,4-Dihydro-2,5,7,8-tetramethyl-6-methoxy-2H-1-benzopyran -2-ylpropandicarbonsäure-diäthylester, 1,68 g Kaliumhydroxyd und 75 ml Äthylenglykol-Wasser, Volumen-Verhältnis 9:1 wird unter Rühren 6 Stunden auf Rückflusstemperatur erhitzt. Das Reaktionsprodukt wird auf Eis gegeben und mit Äther extrahiert, wobei der ätherische Extrakt verworfen wird. Die wässrige Lösung wird mit 3N Salzsäure angesäuert und das gebildete Präzipitat dreimal mit Äther extrahiert. Die kombinierten Ätherextrakte werden mit gesättigter Kochsalzlösung gewaschen, über Magnesiumsulfat getrocknet, filtriert und im Vakuum konzentriert. Die Reinigung des Rückstandes erfolgt durch Dickschichtchromatographie, wobei 0,72 g (75,9%) rac-3,4-Dihydro-6-methoxy-2,5,7,8-tetramethyl-

2H-1-benzopyran-2-essigsäure anfallen und zwar als farbloser Festkörper mit dem Schmelzpunkt von 96–98°C. Die Demethylierung erfolgt unter Verwendung des Prozedere des Beispiels 14, und man gelangt so zur rac-3,4-Dihydro-6-hydroxy-2,5,7,8-tetramethyl-2H-1-benzopyran -2-essigsäure, welch letztere gemäss Helv. Chim. Acta 59, 290 (1976) in das alpha-Tocopherol übergeführt werden kann.

Beispiel 17

rac-2,6-Dimethoxy-3,4-dihydro-2,5,7,8-tetramethyl-2H-1-benzopyran

Ein Gemisch von 200 g (0,847 Mol) rac-3,4-Dihydro-2-methoxy-2,5,7,8-tetramethyl-2H-1-benzopyran -6-ol. 1,2 l Aceton, 200 ml Dimethylsulfat und 100 g Natriumhydroxyd in 100 ml Wasser werden bei Zimmertemperatur 4 Stunden gerührt; es erfolgt Zugabe von 500 ml 10%iger wässriger Ammonhydroxydlösung und das Rühren wird 30 Minuten fortgesetzt. Man extrahiert das Gemisch dreimal mit Äther. Die ätherischen Extrakte werden kombiniert, mit Wasser und mit gesättigter Kochsalzlösung gewaschen, über Magnesiumsulfat getrocknet, filtriert und im Vakuum konzentriert, wobei 238,4 g eines Niederschlages anfallen, welcher aus Äther-Hexan bei –20°C umkristallisiert wird. Man erhält 145,9 g (68,9%) rac-2,6-Dimethoxy-3,4-dihydro-2,5,7,8-tetramethyl-2H-1 -benzopyran als farblosen Niederschlag. Das Produkt liefert nach Umkristallisation in hexan bei –60°C einen Festkörper vom Schmelzpunkt 37–38°C.
Anal. ($C_{15}H_{22}O_3$)
berechnet: C, 71,97; H, 8,86
gefunden: C, 71,84; H, 8,82.

**Patentansprüche für die Vertragsstaaten BE, CH, DE, FR, GB, IT, LI, NL**

1. Verbindungen der Formel

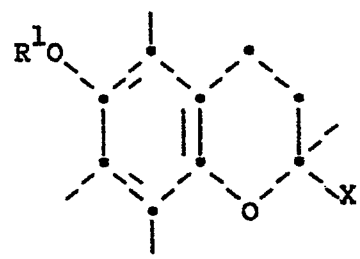

I

worin X Halogen und $R^1$ Methyl oder eine Hydroxy-Schutzgruppe, die durch basische Hydrolyse oder Hydrogenolyse abspaltbar ist, darstellen.
2. Eine Verbindung gemäss Anspruch 1, worin X Chlor darstellt.
3. Eine Verbindung gemäss Anspruch 2, worin $R^1$ Aralkyl darstellt.
4. 2-Chlor-3,4-dihydro-2,5,7,8-tetramethyl-6-(phenylmethoxy)-2H-1-benzopyran.
5. Eine Verbindung gemäss Anspruch 2, worin $R^1$ nieder Alkanoyl darstellt.
6. 2-Chlor-3,4-dihydro-2,5,7,8-tetramethyl-2H-1-benzopyran-6-ol-acetat.
7. 2-Chlor-3,4-dihydro-2,5,7,8-tetramethyl-6-methoxy-2H-1-benzopyran.
8. Eine Verbindung der Formel

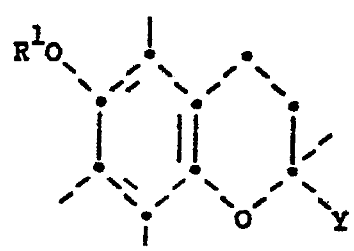

IX

worin Y 2-Propenyl oder das Radikal $-CH(COOR^3)_2$ darstellt, $R^3$ nieder Alkyl ist und $R^1$ Methyl oder eine Hydroxy-Schutzgruppe, die durch basische Hydrolyse oder Hydrierung abspaltbar ist, darstellt.
9. Eine Verbindung gemäss Anspruch 8, worin $R^1$ Aralkyl oder nieder Alkanoyl ist.
10. 3,4-Dihydro-2,5,7,8-tetramethyl-6-(phenylmethoxy)-2H-1-benzopyran-2-ylpropandisäure-dimethylester.
11. 3,4-Dihydro-6-(phenylmethoxy)-2-(2-propenyl)-2,5,7,8-tetramethyl-2H-1-benzopyran.
12. Verfahren zur Herstellung von Verbindungen der Formel

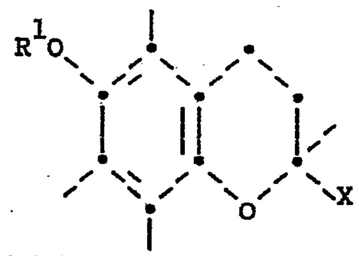

I

worin X Halogen und $R^1$ Methyl oder eine Hydroxy-Schutzgruppe, die durch basische Hydrolyse oder Hydrogenolyse abspaltbar ist, darstellen,
dadurch gekennzeichnet, das man eine Verbindung der Formel

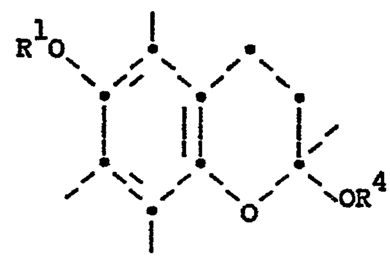

II

worin $R^1$ obige Bedeutung besitzt und $R^4$ Wasserstoff oder nieder Alkyl darstellt,
mit einer Halogenwasserstoffsäure, vorzugsweise in einem inerten organischen Lösungsmittel, vorzugsweise bei Temperaturen von –30°C bis +30°C, umsetzt.

13. Verfahren gemäss Anspruch 12, dadurch gekennzeichnet, dass man die Reaktion unter wasserfreien Bedingungen durchführt.

14. Verfahren gemäss Anspruch 12 oder 13, dadurch gekennzeichnet, dass man als Lösungsmittel einen Äther verwendet.

15. Verfahren zur Herstellung von Verbindungen der Formel

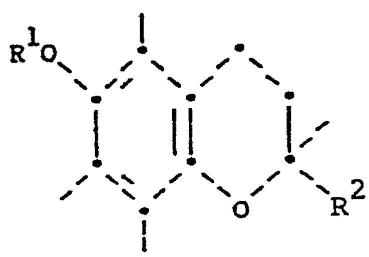

IX

worin $R^1$ Methyl oder eine Hydroxy-Schutzgruppe darstellt, die durch basische Hydrolyse oder Hydrogenolyse abspaltbar ist, $R^2$ die Bedeutungen $-CH(COOR^3)_2$,

$$-CH_2-(CH_2)_2-CH(CH_3)-(CH_2)_3-CH(CH_3)-(CH_2)_3-CH(CH_3)-CH_3$$

oder $-CH_2-CH=CH_2$ hat und $R^3$ nieder Alkyl darstellt,
dadurch gekennzeichnet, dass man eine Verbindung der Formel

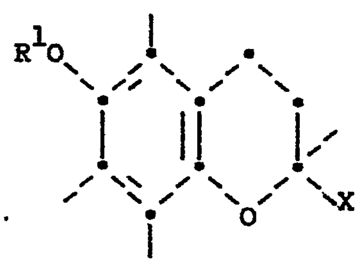

I

worin $R^1$ obige Bedeutung hat und X Halogen darstellt,

mit einer Verbindung der Formel

$R^2MgX$ II

worin $R^2$ und X obige Bedeutung besitzen,
vorzugsweise in einem inerten organischen Lösungsmittel, vorzugsweise bei Temperaturen von –100°C bis 0°C, umsetzt.

16. Verfahren nach Anspruch 15, dadurch gekennzeichnet, dass man Temperaturen von –80°C bis –30°C arbeitet.

17. Verfahren gemäss Anspruch 15 oder 16, dadurch gekennzeichnet, dass man in einem Äther als Lösungsmittel arbeitet.

18. Verfahren zur Herstellung von Verbindungen der Formel

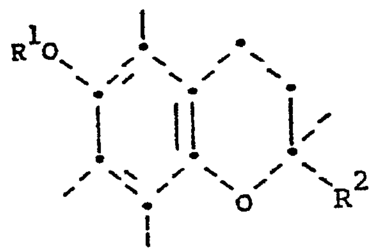

IX

worin $R^1$ Methyl oder eine Hydroxy-Schutzgruppe darstellt, die durch basische Hydrolyse oder Hydrogenolyse abspaltbar ist und $R^2$ die Bedeutungen $-CH(COOR^3)_2$,

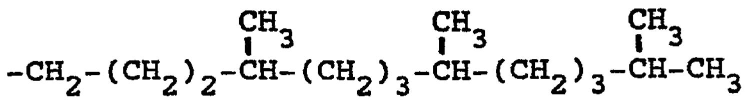

oder $-CH_2-CH=CH_2$ besitzt und $R^3$ nieder Alkyl darstellt,
dadurch gekennzeichnet, dass man eine Verbindung der Formel

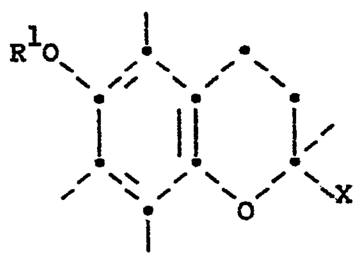

I

worin $R^1$ und X obige Bedeutung besitzen,
mit einer Verbindung der Formel

$R^2M$

worin M ein Alkalimetall ist und $R^2$ obige Bedeutung besitzt,
vorzugsweise in einem inerten organischen Lösungsmittel, vorzugsweise bei Temperaturen von –30°C bis +30°C umsetzt.

19. Verfahren gemäss Anspruch 18, dadurch gekennzeichnet, dass man bei Temperaturen von 0°C bis 15°C arbeitet.

20. Verfahren gemäss Anspruch 18 oder 19, dadurch gekennzeichnet, dass man in einem Äther als Lösungsmittel arbeitet.

21. Verfahren gemäss einem der Ansprüche 15–20, dadurch gekennzeichnet, dass man die erhaltene Verbindung IX nach an sich bekannten Methoden in Vitamin E überführt.

## Patentansprüche für den Vertragsstaat AT

1. Verfahren zur Herstellung von Verbindungen der Formel

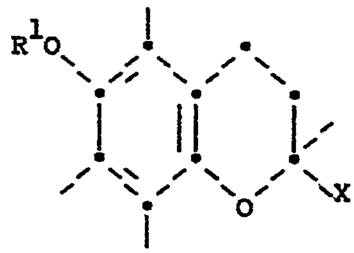

I

worin X Halogen und $R^1$ Methyl oder eine Hydroxy-Schutzgruppe, die durch basische Hydrolyse oder Hydrogenolyse abspaltbar ist, darstellen,
dadurch gekennzeichnet, dass man eine Verbindung der Formel

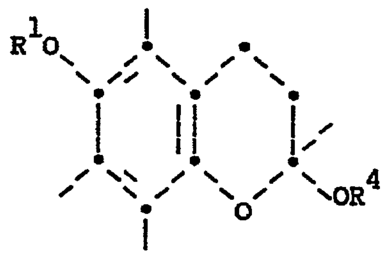

II

worin $R^1$ obige Bedeutung besitzt und $R^4$ Wasserstoff oder nieder Alkyl darstellt,
mit einer Halogenwasserstoffsäure, vorzugsweise in einem inerten organischen Lösungsmittel, vorzugsweise bei Temperaturen von –30°C bis +30°C, umsetzt.

2. Verfahren gemäss Anspruch 1, dadurch gekennzeichnet, dass man die Reaktion unter wasserfreien Bedingungen durchführt.

3. Verfahren gemäss Anspruch 1 oder 2, dadurch gekennzeichnet, dass man als Lösungsmittel einen Äther verwendet.

4. Verfahren zur Herstellung von Verbindungen der Formel

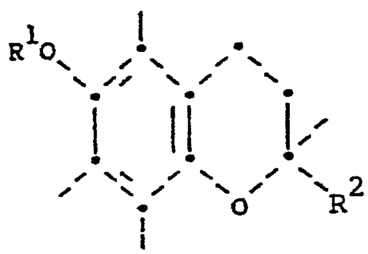

IX

worin $R^1$ Methyl oder eine Hydroxy-Schutzgruppe darstellt, die durch basische Hydrolyse oder Hydrogenolyse abspaltbar ist, $R^2$ die Bedeutungen $-CH(COOR^3)_2$,

$$-CH_2-(CH_2)_2-\overset{\overset{CH_3}{|}}{CH}-(CH_2)_3-\overset{\overset{CH_3}{|}}{CH}-(CH_2)_3-\overset{\overset{CH_3}{|}}{CH}-CH_3$$

oder $-CH_2-CH=CH_2$ hat und $R^3$ nieder Alkyl darstellt,
dadurch gekennzeichnet, dass man eine Verbindung der Formel

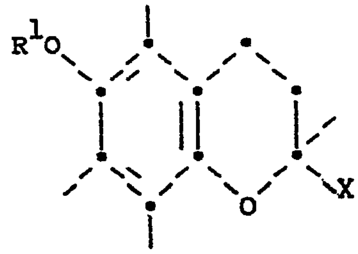

I

worin $R^1$ obige Bedeutung hat und X Halogen darstellt,
mit einer Verbindung der Formel

$R^2MgX$ II

worin $R^2$ und X obige Bedeutung besitzen,
vorzugsweise in einem inerten organischen Lösungsmittel, vorzugsweise bei Temperaturen von –100°C bis 0°C, umsetzt.

5. Verfahren nach Anspruch 4, dadurch gekennzeichnet, dass man bei Temperaturen von –80°C bis –30°C arbeitet.

6. Verfahren gemäss Anspruch 4 oder 5, dadurch gekennzeichnet, dass man in einem Äther als Lösungsmittel arbeitet.

7. Verfahren zur Herstellung von Verbindungen der Formel

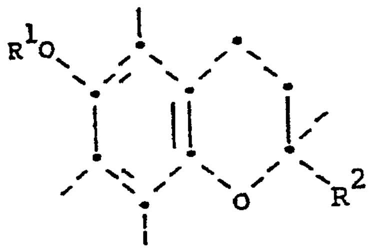

IX

worin $R^1$ Methyl oder eine Hydroxy-Schutzgruppe darstellt, die durch basische Hydrolyse oder Hydrogenolyse abspaltbar ist und $R^2$ die Bedeutungen $-CH(COOR^3)_2$,

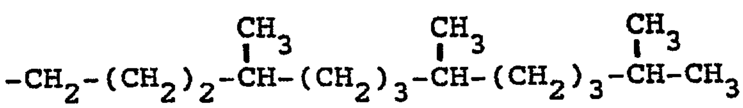

oder $-CH_2-CH=CH_2$ besitzt und $R^3$ nieder Alkyl darstellt,
dadurch gekennzeichnet, dass man eine Verbindung der Formel

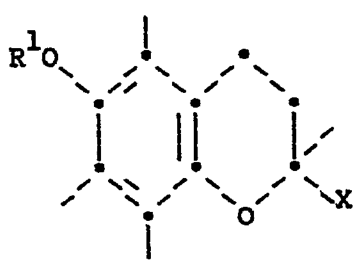

I

worin $R^1$ und X obige Bedeutung besitzen,
mit einer Verbindung der Formel

$R^2M$

worin M ein Alkalimetall ist und $R^2$ obige Bedeutung besitzen,
vorzugsweise in einem inerten organischen Lösungsmittel, vorzugsweise bei Temperaturen von –30°C bis +30°C umsetzt.

8. Verfahren gemäss Anspruch 7, dadurch gekennzeichnet, dass man bei Temperaturen von 0°C bis 15°C arbeitet.

9. Verfahren gemäss Anspruch 7 oder 8, dadurch gekennzeichnet, dass man in einem Äther als Lösungsmittel arbeitet.

10. Verfahren gemäss einem der Ansprüche 4–9, dadurch gekennzeichnet, dass man die erhaltene Verbindung IX nach an sich bekannten Methoden in Vitamin E überführt.

**Claims for the Contracting States BE, CH, DE, FR, GB, IT, LI, NL**

1. Compounds of the formula

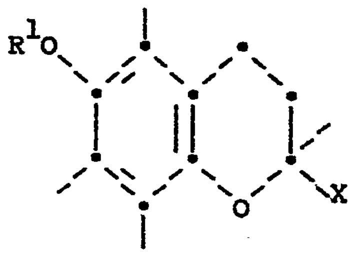

I

wherein X represents halogen and $R^1$ represents methyl or a hydroxy protecting group which is cleavable by basic hydrolysis or hydrogenolysis.

2. A compound according to claim 1, wherein X represents chlorine.

3. A compound according to claim 2, wherein $R^1$ represents aralkyl.

4. 2-Chloro-3,4-dihydro-2,5,7,8-tetramethyl-6-(phenylmethoxy)-2H-1-benzopyran.

5. A compound according to claim 2, wherein $R^1$ represents lower alkanoyl.

6. 2-Chloro-3,4-dihydro-2,5,7,8-tetramethyl-2H-1-benzopyran-6-ol acetate.

7. 2-Chloro-3,4-dihydro-2,5,7,8-tetramethyl-6-methoxy-2H-1-benzopyran.

8. A compound of the formula

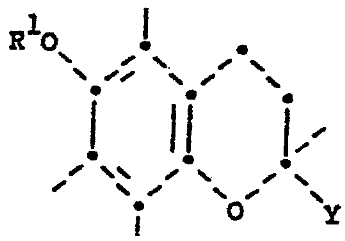

IX

wherein Y represents 2-propenyl or the radical $-CH(COOR^3)_2$, $R^3$ is lower alkyl and $R^1$ represents methyl or a hydroxy protecting group which is cleavable by basic hydrolysis or hydrogenation.

9. A compound according to claim 8, wherein $R^1$ is aralkyl or lower alkanoyl.

10. 3,4-Dihydro-2,5,7,8-tetramethyl-6-(phenylmethoxy)-2H-1-benzopyran-2-ylpropanedioic acid dimethyl ester.

11. 3,4-Dihydro-6-(phenylmethoxy)-2-(2-propenyl)-2,5,7,8-tetramethyl-2H-1-benzopyran.

12. A process for the production of compounds of the formula

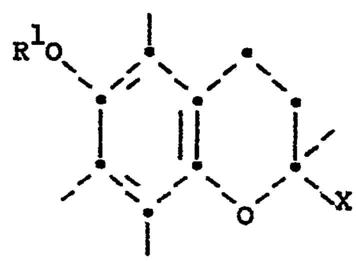

I

wherein X represents halogen and $R^1$ represents methyl or a hydroxy protecting group which is cleavable by basic hydrolysis or hydrogenolysis,
characterized by reacting a compound of the formula

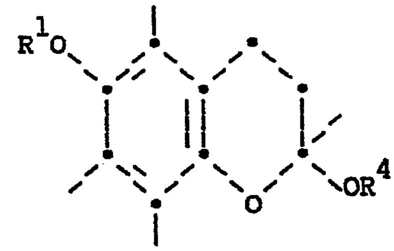

II

wherein $R^1$ has the above significance and $R^4$ represents hydrogen or lower alkyl, with a hydrohalic acid, preferably in an inert organic solvent, preferably at temperatures of –30°C to +30°C.

13. A process according to claim 12, characterized in that the reaction is carried out under anhydrous conditions.

14. A process according to claim 12 or 13, characterized in that an ether is used as the solvent.

15. A process for the production of compounds of the formula

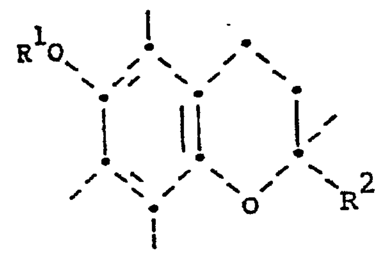

IX

wherein $R^1$ represents methyl or a hydroxy protecting group which is cleavable by basic hydrolysis or hydrogenolysis, $R^2$ has the significances $-CH(COOR^3)_2$,

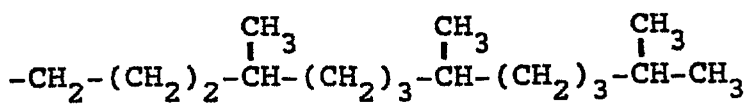

or $-CH_2-CH=CH_2$ and $R^3$ represents lower alkyl, characterized by reacting a compound of the formula

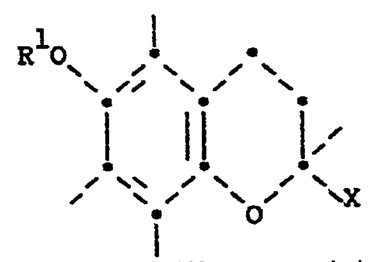

I

wherein $R^1$ has the above significance and X represents halogen, with a compound of the formula

$R^2MgX$ II

wherein $R^2$ and X have the above significance, preferably in an inert organic solvent, preferably at temperatures of –100°C to 0°C.

16. A process according to claim 15, characterized in that the reaction is carried out at temperatures of –80°C to –30°C.

17. A process according to claim 15 or 16, characterized in that the reaction is carried out in an ether as the solvent.

18. A process for the production of compounds of the formula

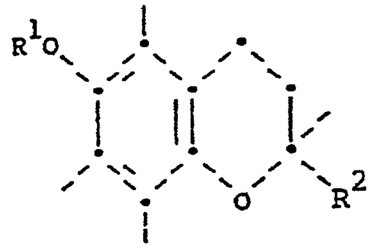

IX

wherein $R^1$ represents methyl or a hydroxy protecting group which is cleavable by basic hydrolysis or hydrogenolysis and $R^2$ has the significances $-CH(COOR^3)_2$,

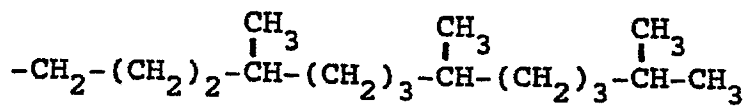

or $-CH_2-CH=CH_2$ and $R^3$ represents lower alkyl,
characterized by reacting a compound of the formula

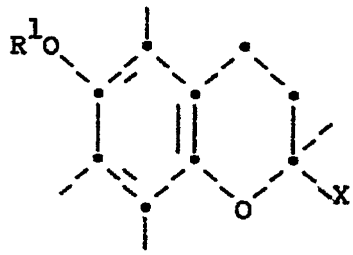

I

wherein $R^1$ and X have the above significance,
with a compound of the formula

$R^2M$

wherein M is an alkali metal and $R^2$ has the above significance,
preferably in an inert organic solvent, preferably at temperatures of –30°C to +30°C.

19. A process according to claim 18, characterized in that the reaction is carried out at temperatures of 0°C to 15°C.

20. A process according to claim 18 or 19, characterized in that the reaction is carried out in an ether as the solvent.

21. A process according to any one of claims 15–20, characterized in that the compound IX obtained is converted into vitamin E according to methods known per se.

**Claims for the Contracting State AT**

1. A process tor the production of compounds of the formula

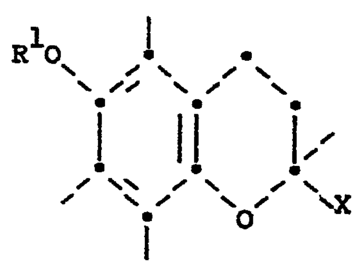

I

wherein X represents halogen and $R^1$ represents methyl or a hydroxy protecting group which is cleavable by basic hydrolysis or hydrogenolysis,
characterized by reacting a compound of the formula

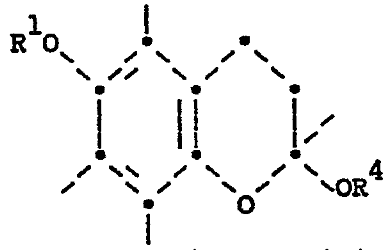

II

wherein $R^1$ has the above significance and $R^4$ represents hydrogen or lower alkyl, with a hydrohalic acid, preferably in an inert organic solvent, preferably at temperatures of –30°C to +30°C.

2. A process according to claim 1, characterized in that the reaction is carried out under anhydrous conditions.

3. A process according ot claim 1 or 2, characterized in that an ether is used as the solvent.

4. A process for the production of compounds of the formula

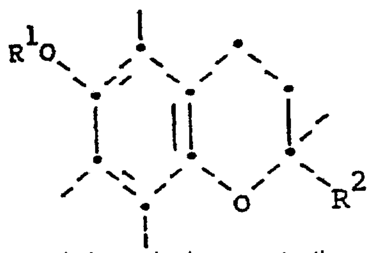

IX

wherein $R^1$ represents methyl or a hydroxy protecting group which is cleavable by basic hydrolysis or hydrogenolysis, $R^2$ has the significances $-CH(COOR^3)_2$,

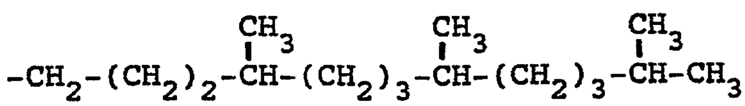

or $-CH_2-CH=CH_2$ and $R^3$ represents lower alkyl, characterized by reacting a compound of the formula

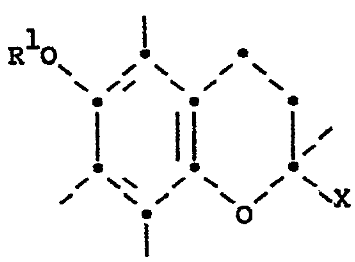

I

wherein $R^1$ has the above significance and X represents halogen, with a compound of the formula

$R^2MgX$    II

wherein $R^2$ and X have the above significance, preferably in an inert organic solvent, preferably at temperatures of –100°C to 0°C.

5. A process according to claim 4, characterized in that the reaction is carried out at temperatures of –80°C to –30°C.

6. A process according to claim 4 or 5, characterized in that the reaction is carried out in an ether as the solvent.

7. A process for the production of compounds of the formula

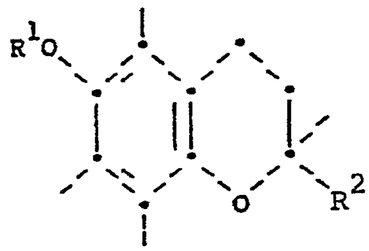

IX

wherein $R^1$ represents methyl or a hydroxy protecting group which is cleavable by basic hydrolysis or hydrogenolysis and $R^2$ has the significances $-CH(COOR^3)_2$,

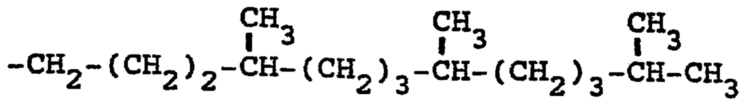

or $-CH_2-CH=CH_2$ and $R^3$ represents lower alkyl, characterized by reacting a compound of the formula

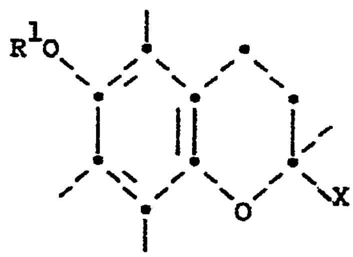

I

wherein $R^1$ and X have the above significance, with a compound of the formula

$R^2M$

wherein M is an alkali metal and $R^2$ has the above significance, preferably in an inert organic solvent, preferably at temperatures of −30°C to +30°C.

8. A process according to claim 7, characterized in that the reaction is carried out at temperatures of 0°C to 15°C.

9. A process according to claim 7 or 8, characterized in that the reaction is carried out in an ether as the solvent.

10. A process according to any one of claims 4–9, characterized in that the compound IX obtained is converted into vitamin E according to methods known per se.

**Revendications pour les Etats contractants BE, CH, DE, FR, GB, IT, LI, NL**

1. Composés de formule

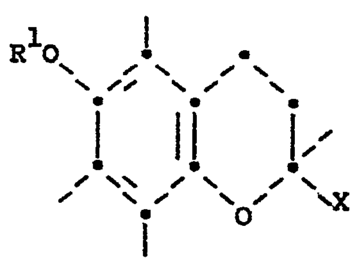

I

dans laquelle X représente un halogène et $R^1$ représente un groupe méthyle ou un groupe protecteur du groupe hydroxy éliminable par hydrolyse basique ou par hydrogénolyse.

2. Un composé selon la revendication 1, dans lequel X représente le chlore.

3. Un composé selon la revendication 2, dans lequel $R^1$ représente un groupe aralkyle.

4. Le 2-chloro-3,4-dihydro-2,5,7,8-tétraméthyl-6-(phénylméthoxy)-2H-1-benzopyranne.

5. Un composé selon la revendication 2, dans lequel $R^1$ représente un groupe alcanoyle inférieur.

6. L'acétate du 2-chloro-3,4-dihydro-2,5,7,8-tétraméthyl-2H-1-benzopyranne-6-ol.

7. Le 2-chloro-3,4-dihydro-2,5,7,8-tétraméthyl-6-méthoxy-2H-1-benzopyranne.

8. Un composé de formule

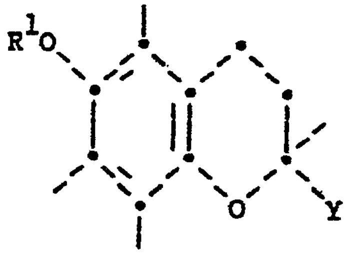

IX

dans laquelle Y représente un groupe 2-propényle ou le radical $-CH(COOR^3)_2$, $R^3$ représente un groupe alkyle inférieur et $R^1$ un groupe méthyle ou un groupe protecteur du groupe hydroxy éliminable par hydrolyse basique ou par hydrogénation.

9. Un composé selon la revendication 8, dans lequel $R^1$ représente un groupe aralkyle ou alcanoyle inférieur.

10. Le 3,4-dihydro-2,5,7,8-tétraméthyl-6-(phénylméthoxy)-2H-1-benzopyranne-2-ylpropane-dioate de diméthyle.

11. Le 3,4-dihydro-6-(phénylméthoxy)-2-(2-propényl)-2,5,7,8-tétraméthyl-2H-1-benzopyranne.

12. Procédé de préparation des composés de formule

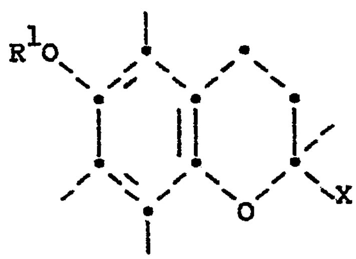

I

dans laquelle X représente un halogène et $R^1$ un groupe méthyle ou un groupe protecteur du groupe hydroxy éliminable par hydrolyse basique ou par hydrogénolyse,
caractérisé en ce que l'on fait réagir un composé de formule

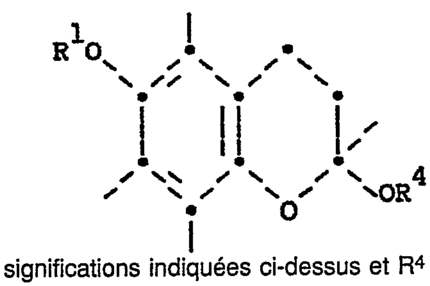

II

dans laquelle $R^1$ a les significations indiquées ci-dessus et $R^4$ représente l'hydrogène ou un groupe alkyle inférieur,
avec un halogénure d'hydrogène, de préférence dans un solvant organique inerte, de préférence à des températures de –30°C à +30°C.

13. Procédé selon la revendication 12, caractérisé en ce que l'on effectue la réaction en milieu anhydre.

14. Procédé selon la revendication 12 ou 13, caractérisé en ce que le solvant utilisé est un éther.

15. Procédé de préparation des composés de formule

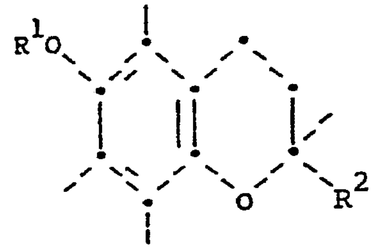

IX

dans laquelle $R^1$ représente un groupe méthyle ou un groupe protecteur du groupe hydroxy éliminable par hydrolyse basique ou par hydrogénolyse, $R^2$ représente $-CH(COOR^3)_2$,

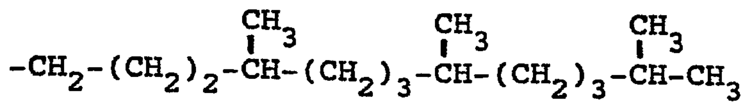

ou $-CH_2-CH=CH_2$ et $R^3$ représente un groupe alkyle inférieur,
caractérisé en ce que l'on fait réagir un composé de formule

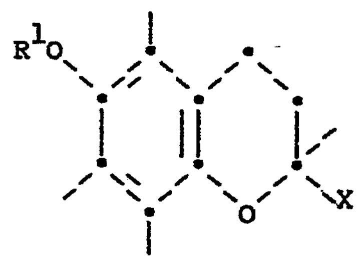

I

dans laquelle $R^1$ a les signification indiquées ci-dessus et X représente un halogène,
avec un composé de formule

$R^2MgX$ II

dans laquelle $R^2$ et X ont les significations indiquées ci-dessus,
de préférence dans un solvant organique inerte, de préference à des températures de −100 à 0°C.

16. Procédé selon la revendication 15, caractérisé en ce que l'on opère à des températures de −80 à −30°C.

17. Procédé selon la revendication 15 ou 16, caractérisé en ce que l'on opère dans un solvant consistant en un éther.

18. Procédé de préparation des composés de formule

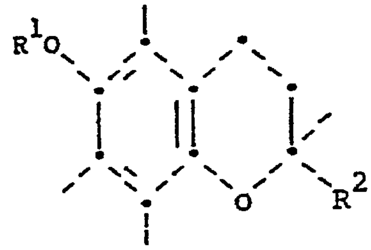

IX

dans laquelle $R^1$ représente un groupe méthyle ou un groupe protecteur du groupe hydroxy éliminable par hydrolyse basique ou par hydrogénolyse et $R^2$ représente $-CH(COOR^3)_2$,

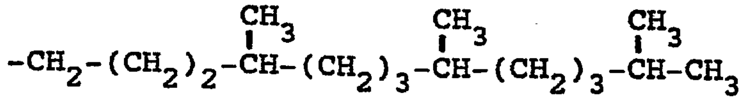

ou $-CH_2-CH=CH_2$ et $R^3$ un groupe alkyle inférieur,
caractérisé en ce que l'on fait réagir un composé de formule

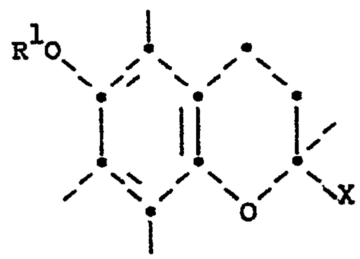

I

dans laquelle $R^1$ et X ont les significations indiquées ci-dessus, avec un composé de formule

$R^2M$

dans laquelle M représente un métal alcalin et $R^2$ a les significations indiquées ci-dessus, de préférence dans un solvant organique inerte, de préférence à des températures de −30 à +30°C.

19. Procédé selon la revendication 18, caractérisé en ce que l'on opère à des températures de 0 à 15°C.

20. Procédé selon la revendication 18 ou 19, caractérisé en ce que l'on opère dans un solvant consistant en un éther.

21. Procédé selon l'une des revendications 15 à 20, caractérisé en ce que l'on convertit le composé IX obtenu, par des procédés connus en soi, en vitamine E.

**Revendications pour l'Etat contractant AT**

1. Procédé de préparation des composés de formule

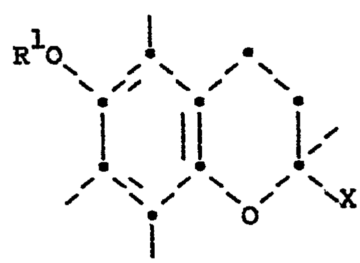

I

dans laquelle X représente un halogène et $R^1$ un groupe méthyle ou un groupe protecteur du groupe hydroxy éliminable par hydrolyse basique ou par hydrogénolyse, caractérisé en ce que l'on fait réagir un composé de formule

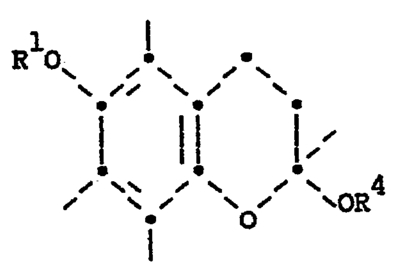

II

dans laquelle $R^1$ a les significations indiquées ci-dessus et $R^4$ représente l'hydrogène ou un groupe alkyle inférieur, avec un halogénure d'hydrogène, de préférence à des températures de −30 à +30°C.

2. Procédé selon la revendication 1, caractérisé en ce que l'on effectue la réaction en milieu anhydre.

3. Procédé selon la revendication 1 ou 2, caractérisé en ce que le solvant utilisé consiste en un éther.

4. Procédé de préparation des composés de formule

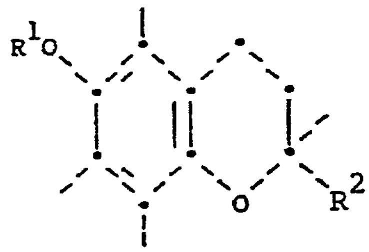

IX

dans laquelle $R^1$ représente un groupe méthyle ou un groupe protecteur du groupe hydroxy éliminable par hydrolyse basique ou par hydrogénolyse, $R^2$ représente $-CH(COOR^3)_2$,

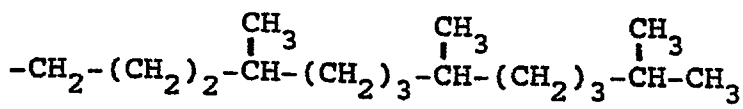

ou $-CH_2-CH=CH_2$ et $R^3$ représente un groupe alkyle inférieur, caractérisé en ce que l'on fait réagir un composé de formule

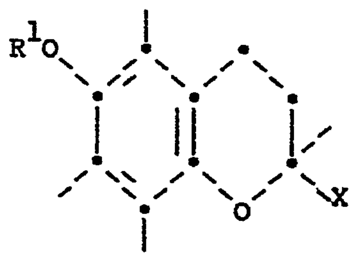

I

dans laquelle $R^1$ a les significations indiquées ci-dessus et X représente un halogène, avec un composé de formule

$R^2MgX$ II

dans laquelle $R^2$ et X ont les significations indiquées ci-dessus, de préférence dans un solvant organique inerte, de préférence à des températures de −100 à 0°C.

5. Procédé selon la revendication 4, caractérisé en ce que l'on opère à des températures de −80 à −30°C.

6. Procédé selon la revendication 4 ou 5, caractérisé en ce que l'on opère dans un solvant consistant en un éther.

7. Procédé de préparation des composés de formule

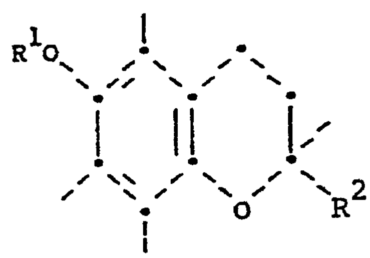

IX

dans laquelle $R^1$ représente un groupe méthyle ou un groupe protecteur du groupe hydroxy éliminable par hydrolyse basique ou par hydrogénolyse et $R^2$ représente $-CH(COOR^3)_2$,

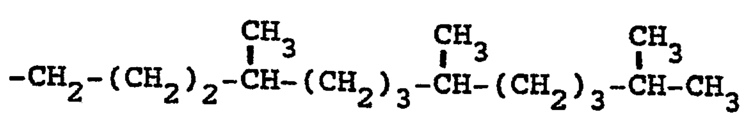

ou $-CH_2-CH=CH_2$ et $R^3$ représente un groupe alkyle inférieur, caractérisé en ce que l'on fait réagir un composé de formule

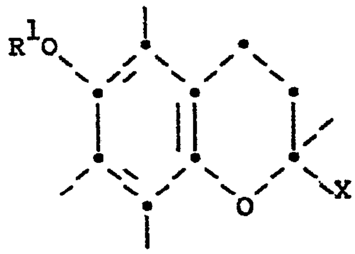

I

dans laquelle $R^1$ et X ont les significations indiquées ci-dessus,
avec un composé de formule

$R^2M$

dans laquelle M représente un métal alcalin et $R^2$ a les significations indiqueées ci-dessus,
de préférence dans un solvant organique inerte, de préférence à des températures de −30 à +30°C.

8. Procédé selon la revendication 7, caratérisé en ce que l'on opère à des températures de 0 à 15°C.

9. Procédé selon la revendication 7 ou 8, caractérisé en ce que l'on opère dans un solvant consistant en un éther.

10. Procédé selon l'une des revendications 4 à 9, caractérisé en ce que l'on convertit le composé IX ainsi obtenu, par des procédés connus en soi, en vitamine E.